# NEW EUROPEAN PATENT SPECIFICATION

(11) **EP 0 507 761 B2**
(45) Date of publication and mention of the opposition decision: **16.04.2003**
(45) Mention of the grant of the patent: 22.03.1995
(21) Application number: 92870056.6
(22) Date of filing: 03.04.1992
(51) Int. Cl.: B01J 29/70, C07C 2/74

(54) **Modified zeolite alkylation catalyst and process employing same**
Modifizierter Zeolit-Alkylierungskatalysator und Gebrauchsverfahren
Zéolite modifiée comme catalyseur d'alkylation et procédé d'utilisation

(30) Priority: 05.04.1991 US 681035
(43) Date of publication of application: 07.10.1992
(73) Proprietor: FINA TECHNOLOGY, INC., Houston, Texas 77267-4412 (US)
(72) Inventor: Shamshoum, Edwar S., Houston, Texas 77062 (US); Schuler, Thomas R., Galena Park, Texas 77547 (US); Ghosh, Ashim K., Houston, Texas 77062 (US)
(74) Representative: Leyder, Francis

(56) References cited:
- EP-A- 0 055 046
- EP-A- 0 159 846
- EP-A- 0 338 734
- EP-A- 0 405 683
- WO-A-91/00777
- US-A- 3 251 897
- US-A- 3 308 069
- US-A- 3 503 874
- US-A- 4 086 186
- US-A- 4 891 458
- WORLD PATENTS INDEX LATEST Week 8743, Derwent Publications Ltd., London, GB; AN87-305992

## Description

This invention relates to an improved, metal-modified, alkylation catalyst and the process for employing such catalyst. The catalyst comprises a lanthanum/beta zeolite which is particularly useful in the alkylation of an aromatic substrate in the liquid phase by a low molecular weight alkylating agent.

### BACKGROUND OF THE INVENTION

Processes for the alkylation of aromatic feedstocks and the use of molecular sieves as catalysts in such alkylation processes are well known in the art. Such alkylation processes may be used to produce mono-/or poly-alkylated products ranging from low to high molecular weights and may be carried out in the vapor phase, in the liquid phase, or under intermediate conditions in which both liquid and vapor phases exist.

U.S. Patent No. 4,301,316 to Young discloses the use of a crystalline zeolite alkylation catalyst in the alkytation of benzene by relatively long chain length alkylating agents having one or more reactive alkyl groups of at least five carbon atoms. The reactants may be in either the vapor phase or the liquid phase, and the zeolite catalyst may be either modified or unmodified. Preferred zeolite catalyst include zeolite beta, ZSM-4, ZSM-20, ZSM-38, and synthetic and naturally occurring isotopes thereof, such as zeolite omega and others. The zeolites may be subjected to various chemical treatments, and may also be subjected to thermal treatments, including steam or calcination in air, hydrogen or an inert gas. Specifically disclosed in Young is the reaction of benzene and 1-dodecene over zeolite beta in a flow reactor at 250°C and 41.37 bar (600psig).

U.S. Patent No. 4,185,040 to Ward et al. discloses an alkylation process employing a molecular sieve catalyst of low sodium content which is said to be especially useful in the production of ethyl benzene from benzene and ethylene, and cumene from benzene and propylene. The Na₂O content of the zeolite should be less than 0.7 weight percent and preferably less than 0.5 weight percent. Examples of suitable zeolites include molecular sieves of the X, Y, L, B, ZSM-5, and Omega crystal types, with steam stabilized hydrogen Y zeolite being preferred. The alkylation process is preferably carried out under conditions in which at least some liquid phase is present, at least until substantially all of the olefin alkylating agent is consumed.

Another alkylation procedure is disclosed in U.S. Patent Nos. 4,798,816 and 4,876,408 to Ratcliffe et al. Ratcliffe et al. employ molecular sieve alkylation catalyst which have been treated in a manner to improve selectivity to monoalkylation, specifically to the propylation of benzene to produce cumene. Selectivity is said to be increased by at least one percentage point by first depositing a carbonaceous material on the catalyst and then subjecting the resultant carbon containing catalyst particles to combustion. Specifically disclosed zeolitic crystalline molecular sieves include those selected from the group of Y zeolites, fluorided Y zeolites, X zeolites, zeolite beta, zeolite L and zeolite omega. The zeolites may be modified to arrive at products of reduced alumina content and reduced sodium content.

Aromatic alkylation reactions such as the alkylation of benzene with ethylene are highly exothermic reactions. As a result the alkylation reactions may be carried out in stages with intermediate cooling steps. For example, U.S. Patent No. 4,107,224 to Dwyer discloses the vapor phase ethylation of benzene over a zeolite catalyst in a down flow reactor with the intermediate injection of cold reactants in a diluent. Specifically disclosed is the interstage injection of ethylene and benzene. Dwyer characterizes the catalyst suitable for use in his invention in terms of those having a constraint index within the approximate range of one to twelve. Suitable zeolites, with the constraint index in parenthesis, are ZSM-5 (8.3), ZSM-11 (8.7), ZSM-12 (2), ZSM-35 (4.5), ZSM-38 (2) and similar materials. Various molecular sieves including, inter alia, zeolite beta (constraint index 0.6), are disclosed as having constraint indices outside of the range suitable for the Dwyer ethylbenzene production process.

U.S. Patent No. 4,891,458 to Innes is directed to a process for the alkylation or transalkylation of an aromatic hydrocarbon with a C₂ to C₄ olefin alkylating agent under at least partial liquid phase conditions utilizing zeolite beta as the catalyst.

### SUMMARY OF THE INVENTION

In accordance with the present invention there is now provided a metal-modified alkylation catalyst and process for employing such catalyst in the alkylation of aromatic substrates with relatively low molecular weight alkylating agents. Although it is preferred that the catalyst be utilized under moderate temperature conditions, including liquid-phase conditions, the catalyst may also be utilized in vapor phase operation. The catalyst comprises a metal-modified zeolite beta, as outlined in claim 1.

The lanthanum/beta catalyst of the present invention is prepared as outlined in claim 5.

In carrying out the process of the present invention, a feedstock containing an aromatic substrate is supplied to a reaction zone and brought into contact with the molecular sieve alkylation catalyst comprising lanthanum/beta zeolite. The lanthanum/beta zeolite preferably has a lanthanum content of approximately 0.1 weight percent and is characterized by a surface area of at least 650m²/gram, based upon the crystalline zeolite beta. A C₂ to C₄ alkylating agent is also supplied to the reaction zone which is operated under temperature and pressure conditions to maintain the aromatic substrate in the liquid phase, while causing alkylation of the aromatic substrate in the presence of the alkylation catalyst. The resulting alkylated aromatic substrate is then recovered from the reaction zone.

An application of the present invention is in the alkylation of benzene with ethylene to produce ethylbenzene. The process is carried out in liquid phase, as noted above, and at alkylation conditions under which the xylene make, based upon the amount of ethylbenzene produced, is no more than 0.03 weight percent. In another embodiment of the invention, liquid-phase alkylation is carried out using a plurality of series connected reaction stages operated at an average temperature of no more than 300°C with the interstage injection of the C₂ to C₄ alkylating agent in a manner to maintain at least one mole percent of alkylating agent solubilized in the aromatic substrate.

### BRIEF DESCRIPTION OF THE DRAWING

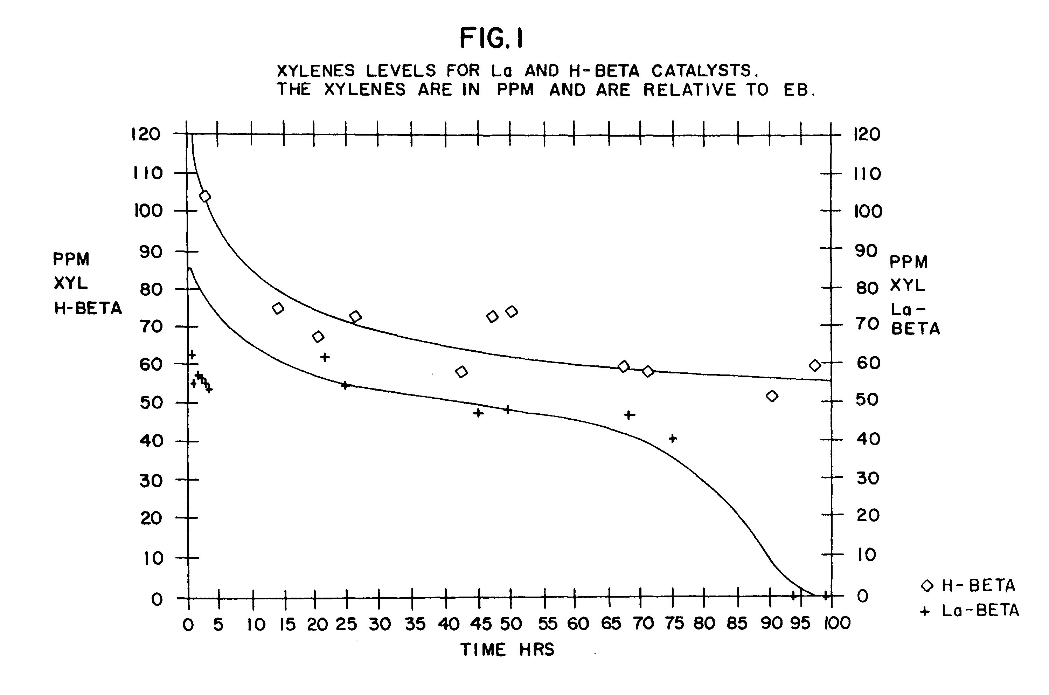

FIGURE 1 is a graph showing the results of experimental work of the alkylation of benzene with ethylene over the catalyst of the present invention as compared to an unmodified zeolite beta.

### DETAILED DESCRIPTION

The present invention relates to an improved alkylation catalyst and the process for employing such catalyst. The catalyst comprises a lanthanum/beta zeolite which is particularly useful in the alkylation of an aromatic substrate by a low molecular weight alkylating agent. The invention is especially applicable to the ethylation of benzene under mild liquid-phase conditions producing little or no xylene make, and the invention will be described specifically by reference to the production of ethylbenzene. However, other alkylating reactions may be utilized in carrying out the invention. For example, the invention may be applied to the reaction of propylene with benzene to produce cumene. Also, while olefinic alkylating agents normally will be employed, other alkylating agents such as alkynes, alkyl halides, alcohols, ethers, and esters as disclosed, for example in U.S. Patent 3,551,510 to Pollitzer et al. may be used. Other aromatic substrates such as toluene and xylene may also be subject to alkylation in accordance with the invention. Further, while the present invention is particularly useful for liquid-phase operations, vapor phase or mixed liquid/vapor phases may also be employed.

It has been previously shown that zeolite beta in its H-form is a very good catalyst for liquid-phase alkylation. The lanthanum/beta catalyst of the present invention, however, exhibits better selectivity to xylene formation than does its hydrogen counterpart.

The lanthanum/beta zeolite of the present invention can be prepared by modification of the crystalline zeolite beta. Basic procedures for the preparation of crystalline zeolite beta which is identified by its x-ray defraction pattern are disclosed in U.S. Patent 3,308,069 to Wadlinger et al. Additionly, European Patent Application 159,846 to Reuben discloses the synthesis of zeolite beta having a silica/alumina mole ratio of up to 300 by employing a templating agent formed by the combination of dimethyl benzyl amine and benzyl halide. Other methods for the preparation of zeolite beta are disclosed in European Patent Application 165,208 to Bruce et al., European Patent Application 186,447 to Kennedy et al., and U.S. Patent No. 4,642,226 to Calvert et al. Each of these procedures is known in the art and suitable for preparing the crystalline zeolite beta useful in the present invention.

The parent zeolite beta catalyst employed in the present invention most preferably is of ultra-low sodium content. Although low sodium content zeolite betas are, in themselves, known in the art (U.S. Patent Nos. 3,308,069 to Wadlinger et al. and 4,642,226 to Calvert et al.), the preferred parent zeolite beta employed in the present invention is characterized by a sodium content substantially lower than those disclosed in the Wadlinger et al. and Calvert et al. patents. Preferably, the parent zeolite beta utilized in the present invention has a sodium content of less than 0.04 weight percent, and most preferably less than 0.02 weight percent, expressed as Na₂O. Such preferred parent zeolite beta is also characterized in terms of a very high surface area, specifically at least 600m²/g based upon the crystalline zeolite beta.

The preferred parent zeolite beta is produced by means of a series of ion-exchange and calcination procedures carried out employing as synthesized zeolite beta as a starting material. The synthesized zeolite beta can be produced by hydrothermal digestion of a reaction mixture comprising silica, alumina, sodium or other alkyl metal oxide, and an organic templating agent in accordance with any suitable procedure such as those disclosed in the aforementioned U.S. patents to Wadlinger et al. and Calvert et al.

Typical digestion conditions include temperatures ranging from slightly below the boiling point of water at atmospheric pressure to about 170°C at pressure equal to or greater than the vapor pressure of water at the temperature involved. The reaction mixture is subjected to mild agitation for periods ranging from about one day to several months to achieve the desired degree of crystallization to form the zeolite beta. Lower temperatures will normally require longer periods in order to arrive at the desired crystal formation. For example, at temperatures of about 100°C crystal growth may occur during periods ranging from about one month to four months, whereas at temperatures near the upper end of the aforementioned range, e.g., about 160°C, the digestion period may be one or two days up to about one week. At intermediate temperatures of 120° to 140°C, the digestion period may extend for about two to four weeks.

Any suitable templating agent may be used in forming the zeolite beta molecular sieve crystalline structure and, as indicated by the references referred to above, appropriate templating agents include tetraethylammonium hydroxides and halides such as tetraethylammonium chloride, dibenzyldimethylammonium hydroxide, or dibenzyldimethylammonium chloride. The reaction components may be varied in accordance with the techniques well known in the art to provide the zeolite beta product of varying silica/alumina ratios. Typically, the reaction mixture used to synthesize the zeolite beta molecular sieve will contain formulations within the following mole ratio ranges:

**TABLE I**

| | |
|---|---|
| SiO₂/Al₂O₃: | 20-1000 |
| H₂O/SiO₂: | 5-200 |
| OH⁻/SiO₂: | 0.1-0.2 |
| M/SiO₂: | 0.01-1.0 |
| R/SiO₂: | 0.1-2.0 |

In Table I, R is the nitroorgano templating agent, e.g., a tetraethylammonium group and M is an alkali metal ion, usually but not necessarily, sodium. For a further description of zeolite beta and methods for its synthesis, recourse may be had to the above patents and patents applications including, specifically, U.S. Patent Nos. 3,308,069 (Wadlinger et al.) and 4,642,226 (Calvert et al.) the entire disclosures of which are incorporated herein by reference.

It is preferred that the initial beta zeolite has a silica to alumina ratio of between 20 to 50. The as synthesized zeolite beta is initially subjected to an ion exchange step employing an ion exchange medium such as an aqueous solution of an inorganic ammonium salt, e.g., normal ammonium nitrate. Following the ion exchange treatment, the zeolite beta is subjected to calcination at a maximum temperature of about 570°C for a period of two or more hours. After the calcination treatment the zeolite beta is cooled and subjected to another ion exchange treatment which may be carried out with the same inorganic ammonium salt as described previously. At the conclusion of the second ion exchange treatment the zeolite beta normally will have a surface area at least twice that of the surface area of the original starting material and a very low sodium content. The sodium content, calculated as Na₂O, normally will be less than 0.04 wt.% and usually less than 0.02 wt.%. Following the second ion exchange treatment an additional, similar ion exchange treatment may also be employed. After drying, lanthanum is then incorporated into the zeolite system by ion-exchange method utilizing a lanthanum salt solution.

The lanthanum/beta zeolite is mixed with a binder such as alumina sol, gamma-alumina or other refractory oxides to produce a mulled lanthanum/beta zeolite-binder mixture. The mixture is then pelletized by any suitable technique such as extrusion and the resulting pellets then dried, At this point, the pelletized binder-lanthanum/beta zeolite product is calcined under conditions sufficient to decompose the ammonium ions on the active site so the lanthanum/beta zeolite is placed in the active LaH-beta form.

In experimental work carried out respecting the present invention, a lanthanum/beta zeolite catalyst was employed in the reaction of ethylene and benzene to produce ethylbenzene. The parent zeolite beta catalyst was prepared by employing a multiple ion exchange and calcination procedure as described previously. The parent beta zeolite had a silica/alumina ratio of about 25, contained tetraethylammonium hydroxide as a retained templating agent, and had an initial surface area of 210 M²/g and a sodium content of about 0.5-1% Na₂O. The initial ammonium ion exchange treatment was done by submersing the zeolite catalyst in an aqueous solution of ammonium nitrate having a normality of 2 at a zeolite to ammonium nitrate ratio of about 2:3. The zeolite beta was submersed in the ion exchange medium under mild agitation at 85°C for a period of two hours. The zeolite beta was then filtered and dried at 110°C for at least two hours. The dried powder was calcined at programmable temperature to a maximum of 570°C to burn the organic template. This calcined powder was then ion-exchanged for two successive ion exchanges with ammonium ions in a similar manner as described above. The surface area of the conclusion of the second ammonium exchange step was 750m²/g. The sodium content was less than .01 wt.% Na₂O.

Lanthanum was incorporated into the zeolite system by ion exchange method. Eighty (80) grams of anhydrons NH₄/Beta zeolite was suspended in deionized water. A salt solution of lanthanum nitrate was prepared by dissolving 1.247 grams of La(NO₃)₃ in deionized water. This salt solution was slowly added to the zeolite suspension at a temperature of about 90°C. The exchange was continued for about 3 hours. This La-exchanged zeolite was filtered, washed and dried at 110°C. The chemical analysis of this powder form (La/NH₄beta-zeolite) is given below and shows a La content of about 0.1 wt.%.
Chemical analysis (anhydrous basis) of La/beta zeolite:

| Sample | Wt% | | | | | SiO₂/Al₂O₃ |
|---|---|---|---|---|---|---|
| | Si | Al | La | Na* | K* | |
| La/NH₄beta | 43.2 | 2.7 | 0.12 | 0 | 0 | 30.9 |

| | | | | | | |
|---|---|---|---|---|---|---|
| *<0.01% | | | | | | |

The powder lanthanum/beta zeolite (66.7 g, anhydrous basis) was mulled with nitric acid treated alumina (21.9 g, anhydrous basis) and extruded into 1/16 inch pellets. The resulting extruded zeolite pellets were then calcined in an oven under air in a programmed temperature to a maximum of 530°C. The LA/NH₄ beta catalyst was thus converted into La³⁺/H-beta (La³⁺=La₂O₃) catalyst as the NH₃ was evolved (NH₄⁺→NH₃ + H⁺] during calcination of the extrudates at 530°C. The final catalyst had following physical properties:
Surface Area=609.1 m²/g
Micropore Area = 325.0 m²/g
Pore Volume=0.13 ml/g
Average Pore Diameter=33.1 A

The experimental work utilizing the lanthanum/beta zeolite was carried out in an upflow reactor heated by a sandbath set at a nominal temperature of 200°C. The reactor contained 9.06 grams of catalyst. The benzene was supplied to the bottom of the reactor at a rate to provide a space velocity (LHSV) of 12.2 hours to the -1 supplied to provide a benzene/ethylene mole ratio of 9.5, with the actual mole ratio varying during the experimental work from about 9.4 to 9.6. For comparative purposes, H-beta zeolite containing no lanthanum was utilized under identical alkylating conditions as the lanthanum/beta catalyst. The results of the foregoing experimental work are set forth below in Table II, and presented graphically in Figure 1. The effluent analysis is set forth in weight percent for benzene and ethylbenzene, and for various other components. The total xylene yields throughout the run are also set forth in Table II and Figure 1.

**Table II**

| TIME, HRS H-BETA | PPM XYLENE RELATIVE TO ETHYLBENZENE | TIME, HR LA-BETA | PPM XYLENE RELATIVE TO ETHYLBENZENE |
|---|---|---|---|
| 1.00 | 192.74 | 0.5 | 85.21 |
| 1.90 | 221.09 | 1.0 | 62.90 |
| 2.90 | 103.43 | 1.5 | 55.18 |
| 20.40 | 67.23 | 2.0 | 56.20 |
| 14.15 | 74.75 | 2.5 | 55.78 |
| 26.15 | 72.45 | 3.0 | 54.50 |
| 42.45 | 57.63 | 21.5 | 62.49 |
| 47.15 | 72.54 | 24.5 | 54.39 |
| 50.00 | 73.67 | 45.0 | 46.94 |
| 67.50 | 58.97 | 49.5 | 47.91 |
| 71.25 | 57.63 | 68.3 | 46.28 |
| 90.50 | 50.73 | 75.0 | 40.07 |
| 97.25 | 59.20 | 94.0 | 0.00 |
| | | 99.0 | 0.00 |

Examination of the experimental data presented in Table II and bearing in mind that, for the most part, the ethylene charge to the reactor was slightly less than 12 percent of the stoichiometrically equivalent amount of benzene, it can be seen that the lanthanum beta zeolite catalyst is highly active and shows good selectivity to ethylbenzene production. The activity of the catalyst remained substantially undiminished after the duration of the experimental work.

In carrying out the invention, the alkylation reaction is carried out at a pressure well above the vapor pressure of the aromatic substrate at the reaction temperature involved in order to ensure that a liquid phase is retained in the reactor. An order to provide a complete liquid-phase reaction, a flooded bed format is used in which the catalyst bed is completely emerged in liquid. This can be readily accomplished using an upflow technique such as used in the experimental work and this usually will be preferred in carrying out the invention. However, downflow flooded bed operation can be accomplished by control of the outlet flow rate to ensure that the catalyst beds are covered by liquid benzene or other aromatic substrate.

Preferably a staged reaction format is employed in order to ensure good solubility of the ethylene (or other alkylating agent) in the benzene (or other aromatic substrate) so that the entire reaction takes place in the liquid phase. In addition, use of multiple stages provides an opportunity for interstage cooling where adiabatic reactors are used or permits the use of several isothermal reaction stages. It is also preferred that the feedstocks be dehydrated prior to use in the present invention.

Having described specific embodiments of the present invention, it will be understood that modification thereof may be suggested to those skilled in the art, and it is intended to cover all such modifications as fall within the scope of the appended claims.

## Claims

1. A molecular sieve catalyst comprising lanthanum/beta zeolite wherein the lanthanum content is between 0.05 and 0.5 weight percent, wherein the surface area of said molecular sieve, based upon the crystalline structure of said lanthanum/beta zeolite, is at least 650 m²/g and wherein said lanthanum/beta zeolite has a sodium content of less than 0.04 weight percent Na₂O.

2. A molecular sieve catalyst as set forth in Claim 1, wherein the lanthanum content is about 0.1 weight percent.

3. A molecular sieve catalyst as set forth in any of Claims 1 to 2, wherein said molecular sieve comprises lanthanum/beta zeolite in combination with a binder and wherein the surface area of said lanthanum/beta zeolite, based upon the composite of said molecular sieve including said binder, is at least 450 m²/g.

4. A molecular sieve catalyst as set forth in any of the preceding Claims, wherein said sodium content is less than 0.02 weight percent Na₂O.

5. A method for the preparation of a lanthanum/beta zeolite as defined in any one of claims 1 to 4 derived by modification of an alkali metal containing zeolite beta synthesized by the hydrothermal digestion of a reaction mixture comprising silica, alumina, an alkali metal oxide, and an organic templating agent, comprising :
(a) treating said synthesized zeolite beta with an ion-exchange medium to protonate a portion of the active sites in said zeolite by exchanging said alkali metal;
(b) calcining under anhydrous conditions said ion-exchanged zeolite at a temperature within the range of 400°-700°C for a period within the range of 2-10 hours;
(c) treating said calcined zeolite with an ion-exchange medium to protonate an additional portion of the active sites in said zeolite by exchanging said alkali metal;
(d) calcining under anhydrous conditions said ion-exchanged zeolite from step (c) at a temperature within the range of 400°-700°C for a period within the range of 2-10 hours;
(e) incorporating lanthanum into said zeolite system by treating the ion-exchanged zeolite from step (d) with an ion-exchange medium comprising a lanthanum salt solution to obtain a lanthanum/beta zeolite;
(f) mixing said lanthanum/beta zeolite with a binder to produce a mulled lanthanum/beta zeolite binder mixture; and
(g) pelletizing said lanthanum/beta zeolite binder mixture and drying the resulting pellets.

6. The method as set forth in Claim 5, further comprising the step of calcining said pellets at a temperature within the range of 520°-580°C for a period of 2-4 hours.

7. The method as recited in any of Claims 5 or 6, wherein the ion-exchange medium employed in step (a) is an aqueous solution of an ammonium salt.

8. The method as recited in any of Claims 5 to 7, wherein the ion-exchange medium employed in step (c) is an aqueous solution of an ammonium salt.

9. The method as recited in any of Claims 5 to 8, wherein the lanthanum salt solution employed in step (e) is an aqueous solution of lanthanum nitrate.

10. A process for the liquid phase alkylation of aromatic compounds, comprising :
(a) supplying a feedstock containing an aromatic substrate into a reaction zone and into contact with a lanthanum/beta zeolite alkylation catalyst wherein the lanthanum content is between 0.05 and 0.5 weight percent, wherein the surface area of said molecular sieve, based upon the crystalline structure of said lanthanum/beta zeolite, is at least 650 m²/g and wherein said lanthanum/beta zeolite has a sodium content of less than 0.04 weight percent Na₂O;
(b) supplying a C₂-C₄ alkylation agent to said reaction zone;
(c) operating said reaction zone at temperature and pressure conditions to maintain said aromatic substrate in the liquid phase causing alkylation of said aromatic substrate by said alkylating agent in the presence of said catalyst; and
(d) recovering alkylated aromatic substrate from said reaction zone.

11. The process as recited in Claim 10, wherein said alkylation catalyst comprises said lanthanum/beta zeolite in combination with a binder and wherein the surface area of said lanthanum/beta zeolite is at least 450 m²/g based upon said catalyst including said binder.

12. The process as recited in any of Claims 10 to 11, wherein said lanthanum/beta zeolite has a sodium content of less than 0.02 weight percent Na₂O.

13. The process as recited in any of Claims 10 to 12, wherein the lanthanum content of said lanthanum/beta zeolite catalyst is about 0.1 weight percent.

14. The process as recited in any of Claims 10 to 13, wherein said aromatic substrate comprises benzene and said alkylating agent is an ethylating agent or a propylating agent.

15. The process as recited in Claim 14, wherein said alkylating agent is an olefin.

16. The process as recited in Claim 15, wherein said alkylating agent is ethylene.

## Patentansprüche

1. Molekülsiebkatalysator, umfassend Lanthan/Beta Zeolith, wobei der Lanthangehalt zwischen 0,05 und 0,5 Gewichtsprozent ist, wobei der Oberflächenbereich des Molekülsiebs, basierend auf der kristallinen Struktur des Lanthan/Beta Zeolithen, mindestens 650 m²/g ist, und wobei der Lanthan/Beta Zeolith einen Natriumgehalt von weniger als 0,04 Gewichtsprozent Na₂O hat.

2. Molekülsiebkatalyator nach Anspruch 1, wobei der Lanthangehalt etwa 0,1 Gewichtsprozent beträgt.

3. Molekülsiebkatalsator nach einem von Ansprüchen 1 bis 2, wobei das Molekülsieb Lanthan/Beta Zeolith in Kombination mit einem Bindemittel umfaßt, und wobei der Oberflächenbereich des Lanthan/Beta Zeolithen, basierend auf dem Composite des Molekülsiebs, das Bindemittel einschließend, mindestens 450 m²/g ist.

4. Molekülsiebkatalysator nach einem der vorhergehenden Ansprüche, wobei der Natriumgehalt geringer als 0,02 Gewichtsprozent Na₂O ist.

5. Verfahren für die Herstellung eines Lanthan/Beta Zeolithen nach einem von Ansprüchen 1 bis 4, abstammend von Modifikation eines Alkalimetall enthaltenden Zeolith Beta, synthetisiert durch die hydrothermale Verdauung einer Reaktionsmischung, umfasend Silica, Aluminiumoxid, ein Alkalimetalloxid und ein organisches Marizenmittel, umfassend:
(a) Behandeln des synthetisierten Zeolith Beta mit einem Ionenaustauschmedium unter Protonieren eines Teils der aktiven Stellen in dem Zeolithen durch Austauschen des Alkalimetalls,
(b) Kalzinieren unter wasserfreien Bedingungen den ionenausgetauschten Zeolithen bei einer Temperatur in dem Bereich von 400° - 700°C für eine Dauer in dem Bereich von 2 - 10 Stunden,
(c) Behandeln des kalzinierten Zeolithen mit einem Ionenaustauschmedium unter Protonieren eines zusätzlichen Teils der aktiven Stellen in dem Zeolithen durch Austauschen des Alkalimetalls,
(d) Kalzinieren unter wasserfeien Bedingungen den ionenausgetauschten Zeolithen aus Stufe (c) bei einer Temperatur in dem Bereich von 400° - 700°C für eine Dauer in dem Bereich von 2 - 10 Stunden,
(e) Einbauen von Lanthan in das Zeolithsystem durch Behandeln des ionenausgetauschten Zeolithen von Stufe (d) mit einem Ionenaustauschmedium, umfassend eine Lanthansalzlösung, unter Erhalten eines Lanthan/Beta Zeolithen,
(f) Mischen des Lanthan/Beta Zeolithen mit einem Bindemittel unter Herstellen einer vermahlenen Lanthan/Beta Zeolith Bindemittel Mischung und
(g) Pelletisieren der Lanthan/Beta Zeolith Bindemittel Mischung und Trocknen der sich ergebenden Pellets.

6. Verfahren nach Anspruch 5, ferner umfassend die Stufe von Kalzinieren der Pellets bei einer Temperatur in dem Bereich von 520°C - 580°C für eine Dauer von 2 - 4 Stunden.

7. Verfahren nach einem von Ansprüchen 5 oder 6, wobei das in Stufe (a) verwendete Ionenaustauschmedium eine wäßrige Lösung eines Ammoniumsalzes ist.

8. Verfahren nach einem von Ansprüchen 5 bis 7, wobei das in Stufe (c) verwendete Ionenaustauschmedium eine wäßrige Lösung eines Ammoniumsalzes ist.

9. Verfahren nach einem von Ansprüchen 5 bis 8, wobei die in Stufe (e) verwendete Lanthansalzlösung eine wäßrige Lösung von Lanthannitrat ist.

10. Verfahren für die Flüssigphasenalkylierung von aromatischen Verbindungen, umfassend
(a) Liefern eines Einsatzmaterials, enthaltend ein aromatisches Substrat, in eine Reaktionszone und in Kontakt mit einem Lanthan/Beta Zeolith Alkylierungskatalysator, wobei der Lanthangehalt zwischen 0,05 und 0,5 Gewichtsprozent ist, wobei der Oberflächenbereich des Molekülsiebs, basierend auf der kristallinen Struktur des Lanthan/Beta Zeolithen, mindestens 650 m²/g ist, und wobei der Lanthan/Beta Zeolith einen Natriumgehalt von weniger als 0,04 Gewichtsprozent Na₂O hat,
(b) Liefern eines C₂ - C₄ Alkylierungsmittels zu der Reaktionszone,
(c) Betreiben der Reaktionszone bei Temperatur- und Druckbedingungen unter Aufrechterhalten des aromatischen Substrats in der flüssigen Phase, Alkylierung des aromatischen Substrats durch das Alkylierungsmittel in der Anwesenheit des Katalysators erzeugend, und
(d) Gewinnen von alkyliertem aromatischen Substrat aus der Reaktionszone.

11. Verfahren nach Anspruch 10, wobei der Alkylierungskatalysator den Lanthan/Beta Zeolithen in Kombination mit einem Bindemittel umfaßt, und wobei der Oberflächenbereich des Lanthan/Beta Zeolithen mindestens 450 m²/g, basierend auf dem Katalysator, das Bindemittel einschließend, ist.

12. Verfahren nach einem von Ansprüchen 10 bis 11, wobei der Lanthan/Beta Zeolith einen Natriumgehalt von weniger als 0,02 Gewichtsprozent Na₂O hat.

13. Verfahren nach einem von Ansprüchen 10 bis 12, wobei der Lanthangehalt des Lanthan/Beta Zeolith Katalysators etwa 0,1 Gewichtsprozent beträgt.

14. Verfahren nach einem von Ansprüchen 10 bis 13, wobei das aromatische Substrat Benzol umfaßt, und das Alkylierungsmittel ist ein Ethylierungs- oder ein Propylierungsmittel.

15. Verfahren nach Anspruch 14, wobei das Alkylierungsmittel ein Olefin ist.

16. Verfahren nach Anspruch 15, wobei das Alkylierungsmittel Ethylen ist.

## Revendications

1. Catalyseur du type à tamis moléculaire comprenant de la zéolithe bêta/lanthane, la teneur en lanthane s'élevant entre 0,05 et 05 % en poids, dans lequel l'aire de surface dudit tamis moléculaire, en se basant sur la structure cristalline de ladite zéolithe bêta/lanthane, s'élève à au moins 650 m²/g et dans lequel ladite zéolithe bêta/lanthane possède une teneur en sodium inférieure à 0,04 % en poids de Na₂O.

2. Catalyseur du type à tamis moléculaire selon la revendication 1, dans lequel la teneur en lanthane s'élève à 0,1 % en poids.

3. Catalyseur du type à tamis moléculaire selon l'une quelconque des revendications 1 à 2, dans lequel ledit tamis moléculaire comprend de la zéolithe bêta/lanthane en combinaison avec un liant, et dans lequel l'aire de surface de ladite zéolithe bêta/lanthane, en se basant sur le composite dudit tamis moléculaire englobant ledit liant, s'élève à au moins 450 m²/g.

4. Catalyseur du type à tamis moléculaire selon l'une quelconque des revendications précédentes, dans lequel ladite teneur en sodium est inférieure à 0,02 % en poids de Na₂O.

5. Procédé pour la préparation d'une zéolithe bêta/lanthane tel que définie dans l'une quelconque des revendications 1 à 4, qui dérive de la modification d'une zéolithe bêta contenant un métal alcalin synthétisée par digestion hydrothermique d'un mélange réactionnel comprenant de la silice, de l'alumine, un oxyde de métal alcalin et un agent organique, comprenant le fait de :
(a) traiter avec un milieu d'échange d'ions ladite zéolithe bêta synthétisée dans le but de fixer un proton sur une portion des sites actifs dans ladite zéolithe par échange dudit métal alcalin ;
(b) calciner, dans des conditions anhydres, ladite zéolithe soumise à un échange d'ions, à une température dans la plage de 400 °C à 700 °C pendant un laps de temps dans la plage de 2 à 10 heures ;
(c) traiter ladite zéolithe calcinée avec un milieu d'échantillon dans le but de fixer un proton sur une portion supplémentaire des sites actifs dans ladite zéolithe par échange dudit métal alcalin ;
(d) calciner, dans des conditions anhydres, ladite zéolithe soumise à un échange d'ions que l'on obtient à l'étape (c), à une température dans la plage de 400 °C à 700 °C pendant un laps de temps dans la plage de 2 à 10 heures ;
(e) incorporer du lanthane dans ledit système de zéolithe en traitant la zéolithe soumise à un échange d'ions que l'on obtient à l'étape (d) avec un milieu d'échantillon comprenant une solution de sel de lanthane pour obtenir une zéolithe bêta/lanthane ;
(f) mélanger ladite zéolithe bêta/lanthane avec un liant pour obtenir un mélange de zéolithe bêta/lanthane et de liant, amené à l'état de pâte, et
(g) transformer en boulettes ledit mélange de zéolithe bêta/lanthane et de liant et sécher les boulettes résultantes.

6. Procédé selon la revendication 5, comprenant en outre l'étape consistant à calciner lesdites boulettes à une température dans la plage de 520 °C à 580 °C pendant un laps de temps de 2 à 4 heures.

7. Procédé selon l'une quelconque des revendications 5 et 6, dans lequel le milieu d'échange d'ions utilisé à l'étape (a) est une solution aqueuse d'un sel d'ammonium.

8. Procédé selon l'une quelconque des revendications 5 à 7, dans lequel le milieu d'échange d'ions utilisé à l'étape (c) est une solution aqueuse d'un sel d'ammonium.

9. Procédé selon l'une quelconque des revendications 5 8, dans lequel la solution de sel de lanthane utilisée à l'étape (e) est une solution aqueuse de nitrate de lanthane.

10. Procédé pour l'alkylation de composés aromatiques en phase liquide comprenant le fait de :
(a) alimenter une zone de réaction avec une matière de charge contenant un substrat aromatique et la mettre en contact avec un catalyseur d'alkylation du type de la zéolithe bêta/lanthane, dans lequel la teneur en lanthane se situe entre 0,05 et 0,5 % en poids, dans lequel l'aire de surface dudit tamis moléculaire, en se basant sur la structure cristalline de ladite zéolithe bêta/lanthane, s'élève à au moins 650 m²/g, et dans lequel ladite zéolithe bêta/lanthane possède une teneur en sodium inférieure à 0,04 % en poids de Na₂O ;
(b) alimenter ladite zone de réaction avec un agent d'alkylation contenant de 2 à 4 atomes de carbone ;
(c) mettre ladite zone de réaction en service dans des conditions de température de pression telles que l'on maintient ledit substrat aromatique en phase liquide pour obtenir une alkylation dudit substrat aromatique via ledit agent d'alkylation en présence dudit catalyseur ; et
(d) récupérer le substrat aromatique alkylé à partir de ladite zone de réaction.

11. Procédé selon la revendication 10, dans lequel ledit catalyseur d'alkylation comprend de la zéolithe bêta/lanthane en combinaison avec un liant, et dans lequel l'aire de surface de ladite zéolithe bêta/lanthane, en se basant sur ledit catalyseur englobant ledit liant, s'élève à au moins 450 m²/g.

12. Procédé selon l'une quelconque des revendications 10 à 11, dans lequel ladite zéolithe bêta/lanthane possède une teneur en sodium inférieure à 0,02 % en poids de Na₂O.

13. Procédé selon l'une quelconque des revendications 10 à 12, dans lequel la teneur en lanthane dudit catalyseur de zéolithe bêta/lanthane représente environ 0,1 % en poids.

14. Procédé selon l'une quelconque des revendications 10 à 13, dans lequel ledit substrat aromatique comprend du benzène et ledit agent d'alkylation est un agent d'éthylation ou un agent de propylation.

15. Procédé selon la revendication 14, dans lequel ledit agent d'alkylation est une oléfine.

16. Procédé selon la revendication 15, dans lequel ledit agent d'alkylation est de l'éthylène.
